# EUROPEAN PATENT APPLICATION

(11) **EP 1 929 933 A2**
(43) Date of publication of application: **11.06.2008**
(21) Application number: 07014110.6
(22) Date of filing: 18.07.2007
(51) Int. Cl.: A61B 1/05, A61B 5/00

(54) **Endoscopic diagnosing system**

(30) Priority: 10.08.2006 JP 2006217875
(71) Applicant: Fujinon Corporation, Saitama-shi, Saitama (JP)
(72) Inventor: Abe, Kazunori, Kita-ku Saitama-shi Saitama (JP)
(74) Representative: Klunker . Schmitt-Nilson . Hirsch

(57) **Abstract**

In an endoscopic diagnosing system, signals are exchanged using radio waves at between an electronic endoscope, an operator's processor unit and a director's processor unit. The director's processor unit has a transmitting section that modulate into and transmit a radio wave an operation input signal inputted through a zooming button of an operation input device, and a video-signal processing section that switches display over between split screen to display endoscopic images separately between a plurality of electronic endoscopes on the director's monitor and full screen to display one endoscopic image fully on the director's monitor.

## Description

### Background of the Invention

### 1. Field of the Invention

The present invention relates to an endoscopic diagnosing system that allows an operator who is present in an examination room to conduct endoscopic diagnosis under the instruction of a director who stays in a direction room.

### 2. Description of the Related Art

In the related art, electronic endoscopes are frequently used for medical diagnoses in the medical field. The electronic endoscope is to be inserted into a subject interior at its insertion tube wherein the insertion tube incorporates an imaging device, such as a CCD, at the tip thereof. By processing at the processor unit the image signal acquired at the CCD, the image of a subject interior (hereinafter, referred to as an "endoscopic image") can be observed on the monitor. The electronic endoscopes and processor units are set up in a plurality of examination rooms demarcated by partitioning, say, a hospital room so that a plurality of subjects can be diagnosed with endoscopes at the same time.

With such an electronic endoscopic system, a less experienced physician, e.g. an intern, is possibly to perform a procedure. In such a case, it is required to determine whether or not endoscopic diagnosis is being conducted correctly. There is proposed a surgical assist system that a direction room is prepared for a well-experienced physician to stay so that he/she can give instructions correctly to the operators (see JP-A-2005-118232, JP-A-2000-271147 and JP-A-2005-111080).

The art, described in JP-A-2005-118232, is directed to a plurality of electronic endoscopes. However, it could not monitor the system overall panoramically because the monitor in the direction room is to display an endoscopic image only from one electronic endoscope. Meanwhile, when the director desires to intensively observe a particular point of an endoscopic image, the operator each time is troublesomely notified to perform zooming.

Meanwhile, in the arts described in JP-A-2000-271147 and JP-A-2005-111080, the monitor in the direction room is to display split on the screen the endoscopic images of from all the electronic endoscopes. Particularly, JP-A-2000-271147 discloses that the endoscopic image can be changed to a desired state by the manual operation of a director. However, it requires time and cost in laying lines between the rooms because of exchanging endoscopic images and director's instructions through a communication line, such as an ISDN, laid between the examination rooms and the direction room. Furthermore, over such a communication line as an ISDN, an endoscopic image cannot be sent freely from deterioration unless a certain measure be taken, e.g. decreasing the frame rate of the endoscopic image, performing compression on the endoscopic image. Particularly, where to send a quality endoscopic image without compression, transfer rate is insufficient over such a communication line as an ISDN thus being less feasible.

### Summary of the Invention

The present invention, made in view of the foregoing problem, aims at providing an endoscopic diagnosing system allowing for monitoring the entire panoramically so that correct directions can be given to the operators.

In order to achieve the foregoing object, the present invention is an endoscopic diagnosing system that allows at least one operator who is present in at least one examination room to conduct endoscopic diagnosis, under instructions of a director who stays in a direction room, the system comprising: (i) a plurality of operator's endoscopic diagnosing units in said at least one examination room, each of the operator's endoscopic diagnosing units including an electronic endoscope that takes an image of a subject interior at a point-under-observation and outputs a video signal, to modulate the video signal into a first radio wave to transmit, an operator's processor unit that receives the first radio wave and demodulates the first radio wave into a video signal in a former form, to generate an endoscopic image from the video signal, and an operator's monitor that displays the endoscopic image generated at the operator's processor unit; and (ii) a director's endoscopic diagnosing unit in the direction room, the director's endoscopic diagnosing unit including a director's processor unit that receives the first radio wave and demodulates the first radio wave into a video signal in a former form, to generate an endoscopic image from the video signal, a director's monitor that displays the endoscopic image generated at the director's processor unit, and an operation input device for the director to operate, wherein the director's processor unit comprises: a transmitting section that modulates an operation input signal, inputted by the operation input device, into a second radio wave to transmit; and a display control section that switches display over between a split-screen display that displays endoscopic images of said plurality of electronic endoscopes on the director's monitor and a full-screen display that displays one of the endoscopic images entirely on the director's monitor.

Each of said plurality of operator's endoscopic diagnosing units preferably further comprises: an operator's information input device that inputs inquiry information of from the operator to the director; and an operator's information output device that outputs instruction information of from the director to the operator, and wherein the director's endoscopic diagnosing unit further comprises: a director's information input device that inputs the instruction information; and a director's information output device that outputs the inquiry information, wherein the electronic endoscope superposes a signal representative of the inquiry information on the first radio wave which the video signal is modulated and then transmits the first radio wave, the transmitting section modulates a signal representative of the instruction information into a third radio wave and then transmits the third radio, and the electronic endoscope and the director's processor unit being to send and receive the first radio wave superposed with the signal representative of the inquiry information and the third radio wave which the signal representative of the instruction information is modulated, according to half-duplex operation.

The director's processor unit preferably comprises a receiving section that receives radio waves from said plurality of electronic endoscopes by a switchover at a constant time interval when the split-screen display is being done, and selectively receives a radio wave from one of said plurality of electronic endoscopes forming a basis of the one of endoscopic images being displayed on full screen when the full-screen display is being done.

The operation input device preferably comprises: a display selecting section that selects an endoscopic image for the full-screen display out of the endoscopic images being displayed split on the screen, and a display change section that changes between the split-screen display and the full-screen display.

The operation input device preferably comprises a zooming section that changes a zoom magnification as to the endoscopic image, and the transmitting section preferably modulates an operation input signal to the zooming section into the radio wave and transmits the radio wave.

### Brief Description of the Drawings

Fig. 1 is a schematic figure showing an arrangement of an endoscopic diagnosing system according to the present invention;
Fig. 2 is a schematic view showing a structure of an operator's endoscopic diagnosing unit;
Fig. 3 is a block diagram showing a configuration of an electronic endoscope;
Fig. 4 is an explanatory figure showing an RF signal superposed thereon with speech and identification signals;
Fig. 5 is a block diagram showing a configuration of the operator's endoscopic diagnosing unit;
Fig. 6 is a schematic view showing an arrangement of a director's endoscopic diagnosing unit;
Fig. 7 is a block diagram showing a configuration of a director's processor unit;
Fig. 8 is a schematic figure showing a structure of an operation input device;
Fig. 9A is an explanatory figure showing display forms on a director's monitor in a state endoscopic images are displayed split on the screen; and
Fig. 9B is an explanatory figure showing display forms on a director's monitor in a state an endoscopic image is displayed fully on the screen.

### Detailed Description of the Invention

An endoscopic diagnosing unit 2 includes, say, four operator's endoscopic diagnosing units 13 set up respectively in four examination rooms 10 where operators are to conduct endoscope diagnoses on subjects, and a director's endoscopic diagnosing unit 13 set up in a direction room 12 where a director 12 stays to gives directions for endoscopic diagnosis to the operators.

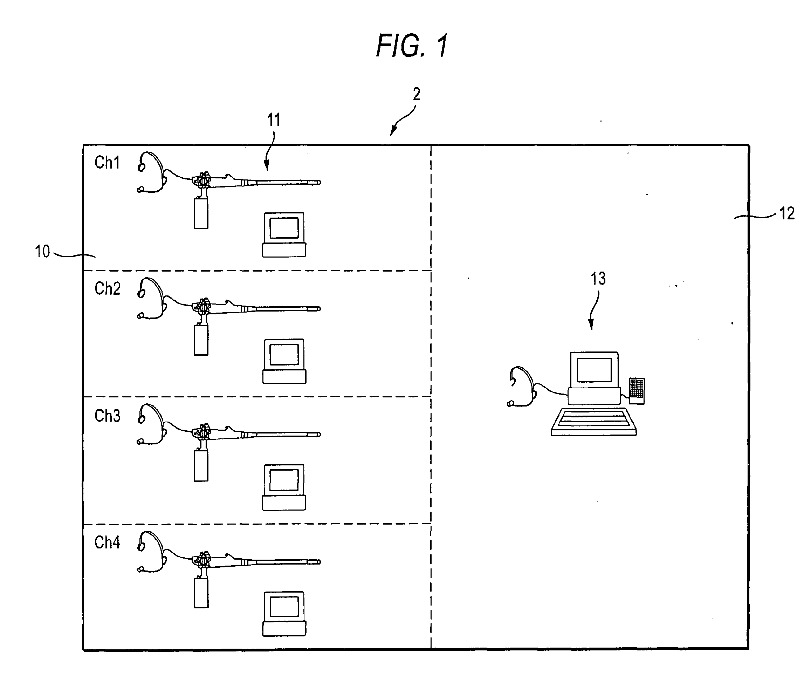

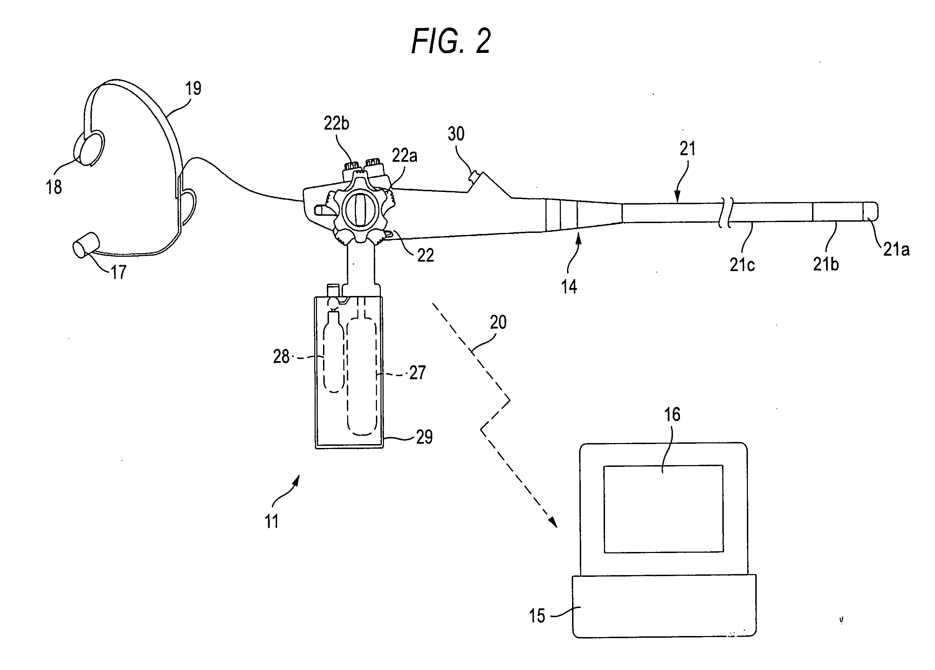

In Fig. 2, the operator's endoscopic diagnosing unit 11 is constructed with an electronic endoscope 14, an operator's processor unit 15 for generating an endoscopic image, an operator's monitor 16 for displaying an endoscopic image, an operator's microphone 17 for capturing an operator's speech, and an operator's headset 19 having an operator's speaker 18 for outputting the operator's speech. The electronic endoscope 14 and the operator's processor unit 15 are to exchange signals by way of radio waves 20. The radio waves 20 have transmission/reception frequency bands that are different between the examination chambers 10, i.e. assigned with four channels (Ch 1 - 4, see Fig. 1).

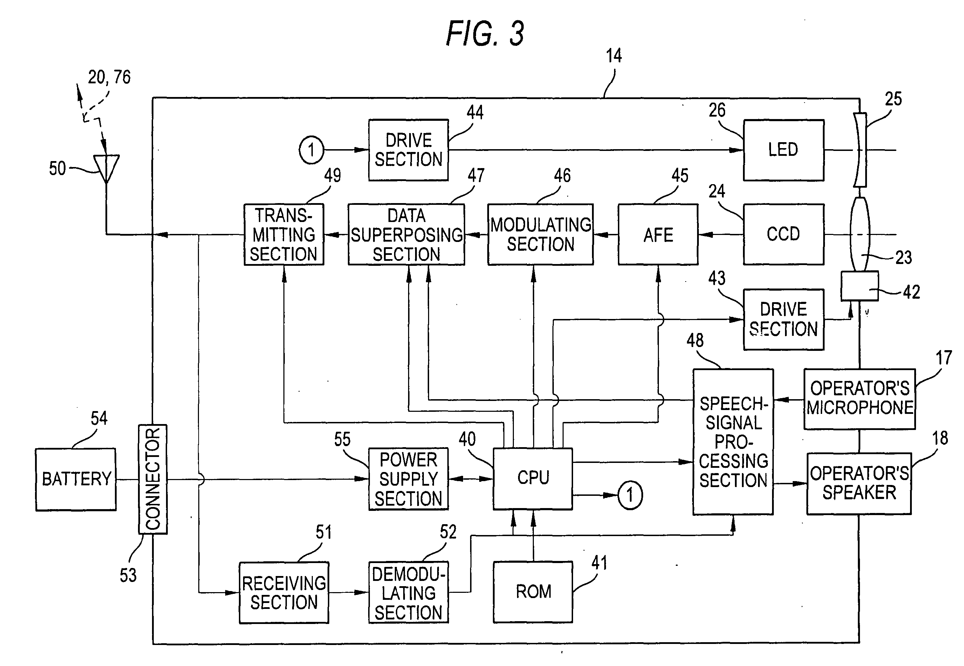

The electronic endoscope 14 has an insertion tube 21 for insertion into a subject body and an operation body 22 provided continuing from the base of the insertion tube 21. The insertion tube 21 has, at its tip end, a tip portion 21a incorporating therein an objective lens 23 for capturing image light from a point-under-observation of a subject body, a CCD 24 for picking up the image light, an illumination lens 25 and an LED 26 for illuminating a subject interior (see Fig. 3, on each).

In rear of the tip portion 21a, a flex portion 21b is provided by a connection of a plurality of flexion pieces. The flex portion 21b is to be bent up, down, left and light by pushing forward and backward a wire passed through the insertion tube 21b through operating an angle knob 22a provided at the operation body 22. This positions the tip portion 21a in a desired direction in a subject interior. In rear of the flex portion 21b, a flexible non-rigid portion 21c is provided to bend in a desired direction along the insertion path into a subject.

Below the operation body 22, a cartridge 29 is removably provided incorporating therein a water reservoir tank 27 storing water and an air bombe 28 storing air. The water and air stored in the water reservoir tank 27 and air bombe 28 is to be ejected to the objective lens 23 through a cleaning nozzle (not shown), formed at the tip portion 21a, through a feed water pipe and feed air pipe arranged within the electronic endoscope 14, in response to the operation of a feed water/air button 21b on the operation body 22. This can remove the dirt, etc. put on the surface of the objective lens 23 and supply air to a subject interior. Here, the cartridge 29 is arranged in a position the palm of an operator abuts upon using the electronic endoscope 14, thus serving also to stabilize the operationality of the electronic endoscope 14. Incidentally, reference character 30 designates a forceps port where to insert a manipulation tool.

In Fig. 3, a CPU 40 is to take total control of the electronic endoscope 14 operation in its entirety. The CPU 40 is connected with a ROM 41 storing the various program and data to control the operation of the electronic endoscope 14. The CPU 40 is to read a required program or data out of the ROM 41 and performs operation control of the electronic endoscope 14.

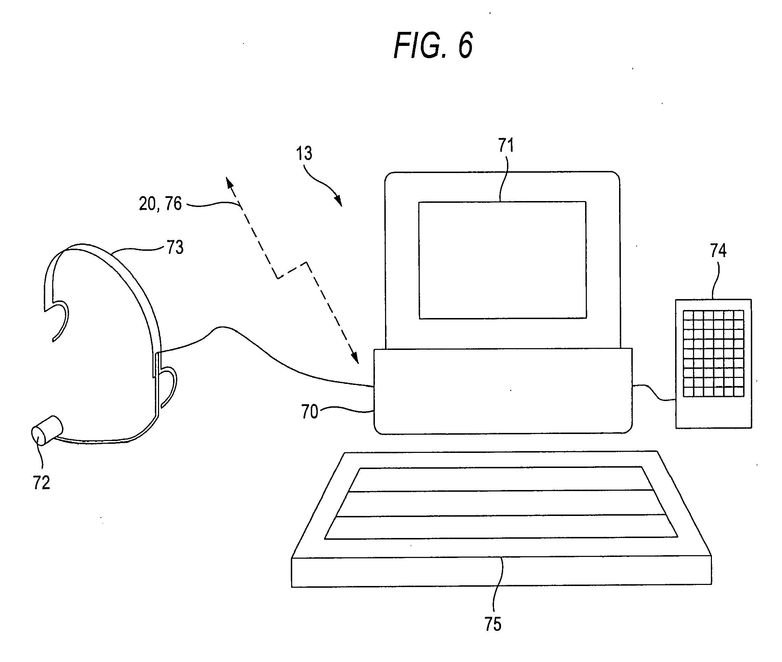

The object lens 23, including a zoom lens (not shown), is connected to a lens motor 42 for moving the zoom lens over its optical axis. The lens motor 42 is placed under operation control of a drive section 43 connected to the CPU 40. The drive section 43 is to operate the lens motor 42 according to an operation input signal of from a zooming button (not shown) provided on the operation body 22 or to an operation input signal sent by way of a radio wave from a director's processor unit 70 (see Fig. 6).

The LED 26 is connected to a drive section 44. The drive section 44 is to on-off drive the LED 26, under control of the CPU 40. The light emitted from the LED 26 is illuminated to a point-under-observation of a subject through the illumination lens 25. Incidentally, the LED 26 may be arranged not in the tip portion 21a but in the operation body 22 so that light can conduct through a light guide to the tip portion 21a.

The CCD 24 is to focus on an image surface, the image light incoming from the point-under-observation of the subject through the objective lens 23, and output an image signal commensurate therewith from pixels thereof. An AFE 45 is to perform correlated double sampling, amplification and A/D conversion on the image signal inputted from the CCD 24, and convert the image signal into a digital video signal under control of the CPU 40.

A modulating section 46 is to perform, say, digital orthogonal modulation on the digital video signal outputted from the AFE 45, and generate an RF signal that is a radiofrequency signal including a video signal and horizontal and vertical synchronization signals. The modulating section 46 is to output the generated RF signal to a data superposing section 47.

A speech-signal processing section 48 is to perform various processes, such as A/D conversion and noise removal, on the operator's speech inputted through the operator's microphone 17, and generate a digital speech signal. The speech-signal processing section 48 is to output the generated speech signal to the data superposing section 47. Meanwhile, the speech-signal processing section 48 is to perform various processes, such as noise removal and A/D conversion, on the speech signal demodulated by a demodulating section 52 as detailed later, and output a processed signal to an operator's speaker 18.

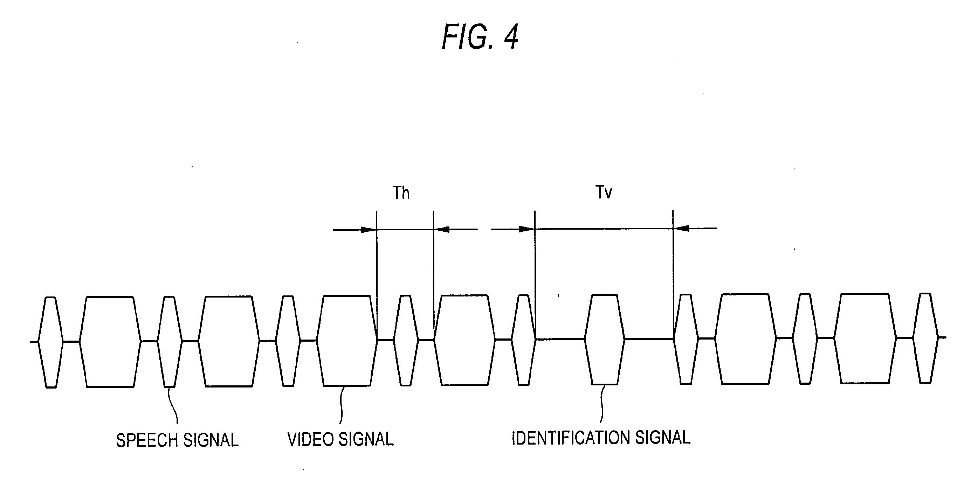

As shown in Fig. 4, the data superposing section 47 is to superpose the speech signal outputted from the speech-signal processing section 48 over a horizontal synchronization period Th of the RF signal generated at the modulating section 46. Meanwhile, the data superposing section 47 is to superpose an identification signal representative of a superposition with speech signal, over a vertical synchronization period Tv portion of the RF signal. When no speeches are inputted to the operator's microphone 17, the data superposing section 47 is not operative. Hence, the RF signal in this case is not superposed with speech and identification signals.

Referring back to Fig. 3, a transmitting section 49 is to convert the RF signal outputted from the data superposing section 47 into a radio wave 20, and send it to the operator's processor unit 15 and director's processor unit 70 by way of an antenna 50 (see Fig. 6).

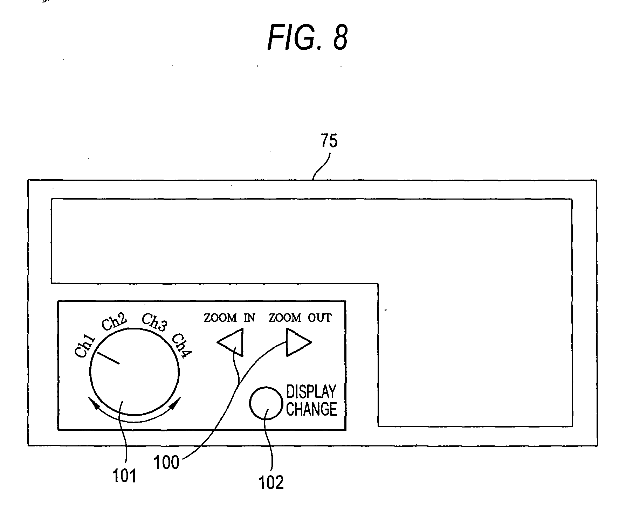

A receiving section 51 is to convert the radio wave 76 (see also Fig. 6), received at the antenna 50 and sent from the director's processor unit 70, into an RF signal and to amplify it. The demodulating section 52 is to perform, say, digital orthogonal detection on the RF signal converted from the radio wave 76, and demodulate the speech signal superposed on the radio wave 76 and the operation input signal to the zooming button 100 (see Fig. 8). The demodulating section 52 is to output the demodulated speech signal and operation input signal to the speech-signal processing section 48 and CPU 40.

A battery 54 is connected to a connector 53. The power of the battery 54 is supplied to the various sections of the electronic endoscope 14 from the power supply section 55 that is under control of the CPU 40. Incidentally, although not shown in Fig. 2, a battery chamber is provided in the rear of the operation body 22 in order to receive a battery 54 therein. The connector 53 is arranged in the chamber. Meanwhile, there is also provided a connector to which the operator's headset 19 is to be connected.

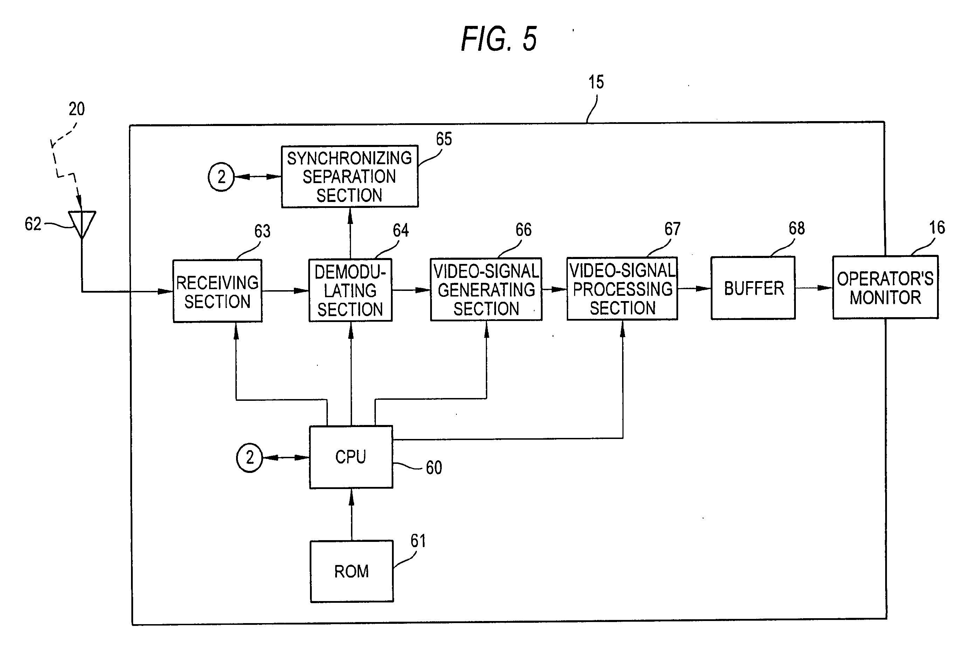

In Fig. 5, a CPU 60 is to take total control of the operator's processor unit 15 operation in its entirety. The CPU 60 is connected with a ROM 61 storing the various programs and data to control the operation of the operator's processor unit 15. The CPU 60 is to read a required program or data out of the ROM 61 and takes operation control of the operator's processor unit 15.

An antenna 62 is to receive a radio wave 20 from the electronic endoscope 14. A receiving section 63 is to convert the radio wave 20 received at the antenna 62 into an RF signal, and amplify it. A demodulating section 64 performs, say, digital orthogonal detection on the RF signal converted from the radio wave 20, thus demodulating the RF signal into a video signal in the form before modulated by the endoscope 14.

Under control of the CPU 60, a synchronization separating section 65 is to separate a synchronization signal out of the video signal demodulated at the demodulating section 64 by means of amplitude-based separation, and then separate horizontal and vertical synchronization signals therefrom by means of frequency-based separation. A video-signal generating section 66 is to generate a digital video signal from the video signal. A video-signal processing section 67 is to perform various signal processes, such as mask generation and character-information addition, on the video signal generated at the video-signal generating section 66. A buffer 68 is to once store the video signal that has been subjected to various signal processes at the video-signal processing section 67 and is to be displayed as an endoscopic image on the operator's monitor 16.

In Fig. 6, the director's endoscopic diagnosing unit 13 is constructed with a director's processor unit 70 for generating an endoscopic image, a director's monitor 71 for displaying an endoscopic image, a director's headset 73 having a director's microphone 72 for capturing the speech of a director, a director's speaker 74 for outputting the speech of a director, and an operation input device 75. The director's processor unit 70 is to exchange signals with the electronic endoscope 14 by way of radio waves 20, 76. The radio wave 70 has a transmission/reception frequency band similar to that of the radio wave 20, which is to be switched over between four channels Ch 1 - 4.

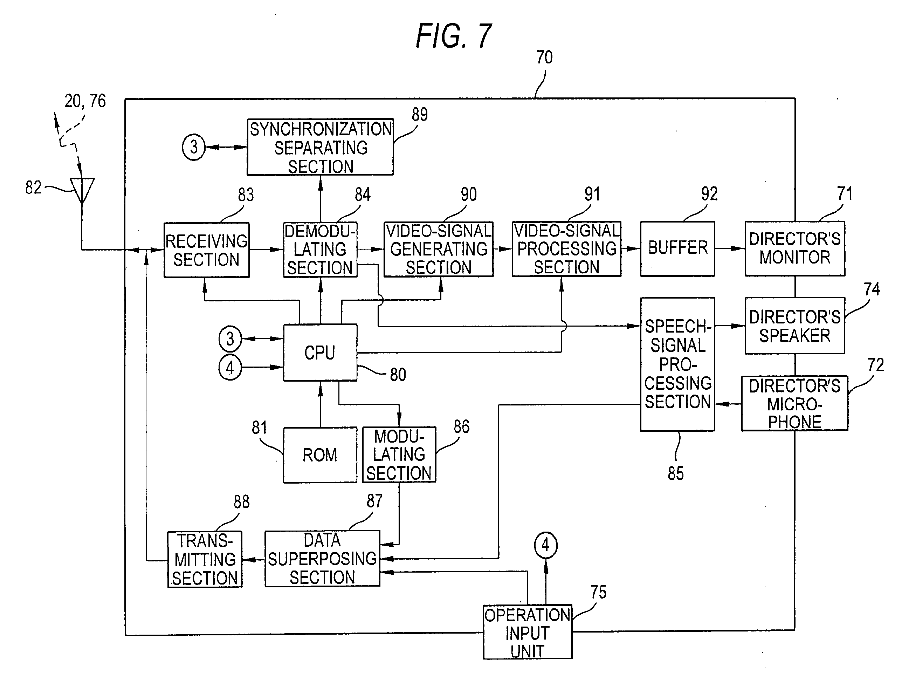

In Fig. 7, a CPU 80 is to take total operation control of the director's processor unit 70 operation in its entirety. The CPU 80 is connected with a ROM 81 storing the various programs and data to control the operation of the director's processor unit 70. The CPU 80 is to read a required program and data out of the ROM 81 and take operation control of the director's processor unit 70.

An antenna 82 is to receive a radio wave 20 from the electronic endoscope 14 and send a radio wave 76 to the electronic endoscope 14. A receiving section 83 is to convert the radio wave 20 received at the antenna 82 into an RF signal and amplify it. A demodulating section 84 is to perform, say, digital orthogonal detection on the RF signal and demodulate the RF signal into a video signal in the form before modulated by the electronic endoscope 14. Meanwhile, the demodulating section 84 is to demodulate the speech signal superposed on the radio wave 20.

A speech-signal processing section 85 is to perform various processes, such as A/D conversion and noise removal, on the director's speech inputted from the director's microphone 72, and output a digital speech signal. The speech-signal processing section 85 is, also, to perform various processes, such as noise removal and A/D conversion, on the speech signal demodulated by a demodulating section 84, and output a processed signal to the director's speaker 74.

A modulating section 86 is to generate an RF signal which a dummy signal is applied to a video signal portion in Fig. 4. A data superposing section 87 is to superpose the speech signal outputted from the speech-signal processing section 85 and the operation input signal onto the zoom-operation button 100, over the horizontal synchronization period Th portion of the RF signal generated at the modulating section 86. Meanwhile, the data superposing section 87 is to superimpose an identification signal representative of a superposition with speech and operation-input signals, over the vertical synchronization period Tv portion of the RF signal. The data superposing section 87 is not operative when there is no input of a speech to the director's microphone 72 or when the zooming button 100 is not pressed. Incidentally, the identification signals superposed at the data superposing sections 47, 87 are mutually different in value in order to be distinguished from each other.

A transmitting section 88 is to convert an RF signal, outputted from the data superposing section 87, into a radio wave 76 and send it to the operator's processor unit 15 by way of an antenna 82. Incidentally, the elements of from the synchronization separating section 89 to the buffer 92 are similar in function to the counterpart elements of from the synchronization separating section 65 to the buffer 68 of the operator's processor unit 15, and hence omitted to explain.

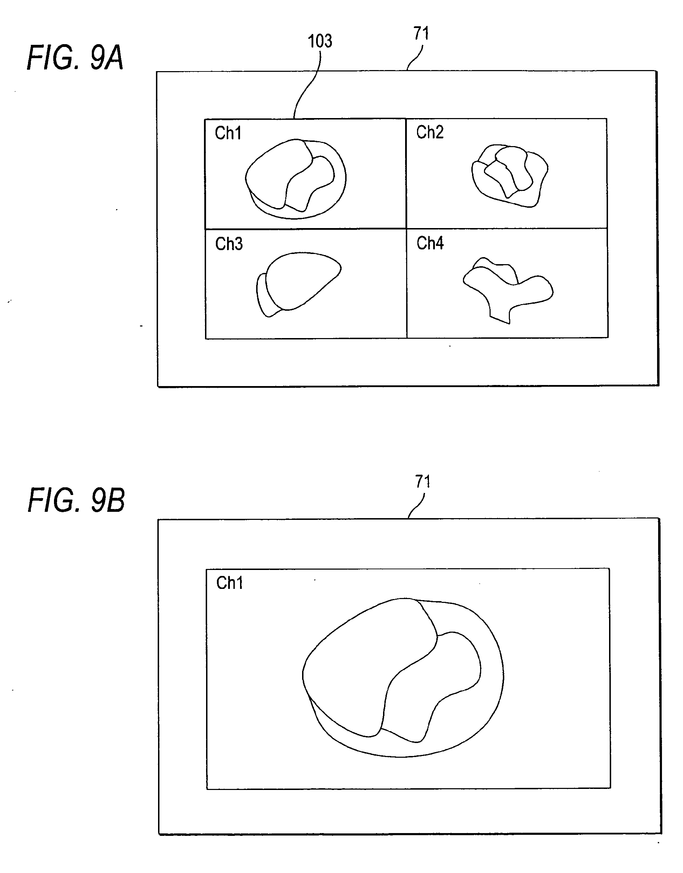

In Fig. 8, the operation input device 75 has a zooming button 100, a channel selector dial 101 and a display change button 102. The zooming button 100 is operated to change the zoom magnification of an endoscopic image being displayed fully on the screen. The channel selector dial 101 is operated to select a channel for the electronic endoscope 14 at which a speech is made effective on the split-screen display (see Figs. 9A and 9B) or a channel for the electronic endoscope 14 at which a radio wave 20 is given off to constitute an endoscopic image to be displayed fully on the screen (see Figs. 9A and 9B). The display change button 102 is operated to change over between split-screen display and full-screen display.

As shown in Figs. 9A and 9B, the video-signal processing section 91 switches over the endoscopic image on display, i.e. endoscopic images separately between four electronic endoscopes 14 on the director's monitor 71 as shown in Fig. 9A, and display on the full screen, i.e. an endoscopic image only based on the electronic endoscope 14 whose channel is selected by the channel selector dial 101 as shown in Fig. 9B, in response to the operation on the display change button 102. Incidentally, where there is an endoscopic diagnosing unit 11 not used, no display is made or a message is displayed indicative of no use, etc. on the relevant area of the split screen.

When split screen is selected by the display change button 102, the receiving section 83 receives radio waves 20 from the four electronic endoscopes 14 while switching over between those at a constant time interval. Meanwhile, when full screen is selected, a radio wave 20 is selectively received from the electronic endoscope 14 whose channel is selected by the channel selector dial 101. Namely, the endoscopic images on the split screen are given as an intermittent moving image having a frame rate commensurate with the switchover interval at the receiving section 83 while the endoscopic image on the full screen is given as a moving image having a frame rate equal to that shown on the relevant operator's monitor 16.

When split screen is selected, the endoscopic image whose channel is selected by the channel selector dial 101 is framed by a bold-lined cursor 103. As for speech, the channel selected by the channel selector dial 101 only is made effective so that the director's speaker 74 outputs the speech inputted to the operator's microphone 17. Meanwhile, at other channels not selected, no output is made effective through the director's speaker 74 even if a speech is input to the operator's microphone 17. The speech-input/output state is taken over even when full screen is selected, thus making effective only the operator's speech whose channel is selected by the channel selector dial 101. Those at the other channels are rendered ineffective.

Meanwhile, the transmitting section 88 sends a radio wave 76 to the operator's processor unit 15 whose channel 15 is selected by the channel selector dial 101. Namely, when split screen is selected, speech is effective only at the channel selected by the channel selector dial 101 so that the operator's speaker 19 outputs the speech inputted to the director's microphone 72. At the other channels not selected, a speech even if inputted to the director's microphone 72 is ineffectively outputted through the operator's speaker 19. Meanwhile, the speech input/output state is taken over even when full screen is selected. This makes effective only the director's speech whose channel is selected by the channel selector dial 101, making ineffective those at the other channels. Furthermore, when split screen is selected, even if the zooming button 100 is operated, the lens motor 42 is ineffectively driven. Zooming is effective only at the channel selected by the channel selector dial 101 with full screen selected. In other words, communication is available between the operator and the director only at the channel selected by the channel selector dial 101.

The electronic endoscope 14 and the director's processor unit 70 exchanges a radio wave 20 superposed with a speech signal and a radio wave 76 superimposed with speech and operation-input signals, according to half-duplex operation. Specifically, the CPU 40 of the electronic microscope 14 detects whether or not an identification signal is superposed on the RF signal converted at the receiving section 51. When an identification signal has been superposed at the data superposing section 87 (when a speech has been inputted to the director's microphone 72 and a speech signal has been superposed on a radio wave 76 or when the zooming button 100 has been operated and an operation input signal has been superposed on a radio wave 76), the data superposing section 47 is controlled in operation not to superpose a speech signal on the RF signal even if there is a speech input to the operator's microphone 17.

When a speech is inputted to the director's microphone 72 and a speech signal is superposed on a radio wave 76 or when the zooming button 100 is operated and an operation input signal is superposed on a radio wave 76, the CPU 80 of the director's processor unit 70 controls the speech-signal processing section 85 not to output an operator's speech through the director's speaker 74 even if receiving a radio wave 20 superposed with a speech signal. Otherwise, it is detected whether or not an identification signal is superposed on the RF signal converted at the receiving section 83. In the case an identification signal has been superposed at the data superposing section 47, the transmitting section 88 is controlled not to send a radio wave 76 even if a speech is inputted to the director's microphone 72 or even if the zooming button 100 is operated. Incidentally, the radio wave 76 is received also at the operator's processor unit 15, the operator's processor unit 15 is adapted not to process but to erase the RF signal whose identification signal has been detected. Processing is made only on the RF signal which is a conversion of the radio wave 20.

When observing a subject interior by use of the endoscopic diagnosing system 2 thus constructed, the LED 26 is turned on. By inserting the insertion tube 21 in a body cavity, the endoscopic image through the CCD 24 is observed on the operator's monitor 16 while illuminating the body cavity.

At this time, the image light of the point-under-observation of the subject, incoming through the objective lens 23, is focused on the image surface of the CCD 24 so that CCD 24 outputs an image signal. The AFE 45 performs a correlated double sampling, amplification and A/D conversion on the image signal outputted from the CCD 24 and converts it into a digital video signal.

The video signal, outputted from the AFE 45, is subjected to digital orthogonal modulation by the modulating section 46, to generate an RF signal. The generated RF signal is amplified at the transmitting section 49 and then sent as a radio wave at the antenna 20.

In the operator's processor unit 15, when the radio wave 20 is received at the antenna 62, the receiving section 63 converts the RF signal into an RF signal and amplifies the converted RF signal. The demodulating section 64 performs digital orthogonal detection on the amplified RF signal and demodulates it into a video signal in the form before modulated by the electronic endoscope 14.

The video signal, demodulated by the demodulating section 64, is subjected to synchronizing separation by the synchronizing separation circuit 65 under control of the CPU 60 so that the video-signal generating section 66 can output a digital video signal. The video signal, outputted from the video-signal generating circuit 66, is subjected to various image processes at the video-signal processing circuit 67 and once stored in the buffer 68, thus being displayed as an endoscopic image on the operator's monitor 16.

In the director's processor unit 70, an endoscopic image is generated from the radio wave 20 similarly to the operator's processor unit 15. The generated endoscopic image is displayed on the director's monitor 71. At this time, in the case split screen has been selected by operating the display change button 102, the receiving section 83 receives radio waves 20, in order, from the four electronic endoscopes 14 at a constant time interval. The director's monitor 71 displays separately endoscopic images based on the four electronic endoscopes 14. The endoscopic image, selected by the channel selector dial 101, is framed with a cursor 103. This allows the director to positively grasp the situation of endoscopic diagnosis being conducted in each examination room 10.

Meanwhile, in the case full screen has been selected by operating the display change button 102, the receiving section 83 receives only a radio wave 20 fom the electronic endoscope 14 whose channel is selected by the channel selector dial 101. The director's monitor 71 displays only an endoscopic image based on the electronic endoscope 14 whose channel is selected by the channel selector dial 101. This allows the director to grasp, in detail, the situation of endoscopic diagnosis being conducted in each examination room 10.

Now explanation is made on the process where the operator on the electronic endoscope 14, whose channel selected by the channel selector dial 101, makes a speech input of an inquiry for director's instruction to the operator's microphone 17. The operator's speech, inputted to the operator's microphone 17, is subjected to various processing at the speech-signal processing section 48 thus being converted into a digital speech signal. In the data superposing section 47, the speech signal, digitized at the speech-signal processing section 48, is superposed on the horizontal synchronization period Th portion of the RF signal of from the modulating section 46. On this occasion, the data superposing section 47 superposes an identification signal on the vertical synchronization period Tv portion of the RF signal. The RF signal, thus superposed with the speech and identification signals, is converted into a radio wave 20 through the transmitting section 49 and antenna 50 and then sent to the operator's processor unit 15 and director's processor unit 70.

In the operator's processor unit 15, an endoscopic image is generated from the radio wave 20 according to the foregoing process. Meanwhile, in the director's processor unit 70, when the radio wave 20 is received at the antenna 82, an RF signal represented by the radio wave 20 is inputted to the demodulating section 84 via the receiving section 83. The demodulating section 84 demodulates it into the former form of video and speech signals.

The demodulated video signal is turned into an endoscopic image through the processing similar to that of the operator's processor unit 15, and displayed fully on the screen of the director's monitor 71. Meanwhile, the demodulated speech signal is subjected to various processes at the speech-signal processing section 85 and outputted to the director's speaker 74. This allows the operator to make an inquiry to the director by use of the operator's microphone 17. The director can listen on the inquiry of from the operator, by use of the director's speaker 74.

Now explanation is made on the process where the director makes a speech input of an instruction for endoscopic diagnosis to the director's microphone 72. The director's speech, inputted to the director's microphone 72, is subjected to various processes at the speech-signal processing section 85 and converted into a digital speech signal. The speech signal, digitized at the speech-signal processing section 85, is superposed on the horizontal synchronization period Th portion of a. dummy video signal generated at the modulating section 86 in a data superposing section 87 and sent as a radio wave 76 to the electronic endoscope 14 whose channel is selected by the channel selector dial 101 by way of the transmitting section 88 and the antenna 82.

In the electronic endoscope 14, when the radio wave 76 is received at the antenna 62, an RF signal represented by the radio wave 76 is inputted to the demodulating section 52 via the receiving section 51. The demodulating section 52 demodulates it into the former dummy form of video and speech signals.

The demodulated speech signal is subjected to various processes at the speech-signal processing section 48 and outputted to the operator's speaker 18. The dummy video signal is erased away without being processed. This allows the director to give an instruction to the operator by use of the director's microphone 72. The operator can listen on the director's instruction through the operator's speaker 18.

Now explanation is made on the process where the director operates the zooming button 100 in the state that full screen is selected. The operation input signal to the zooming button 100 is outputted to the data superposing section 87 where it is superposed on the horizontal synchronization period Th portion of a dummy video signal generated at the modulating section 86 and sent as a radio wave 76 to the electronic endoscope 14 whose channel is selected by the channel selector dial 101 by way of the transmitting section 88 and the antenna 82.

In the electronic endoscope 14, when the radio wave 76 is received-at the antenna 62, an RF signal represented by the radio wave 76 is inputted to the demodulating section 52 via the receiving section 51. The demodulating section 52 demodulates it into the former dummy form of video and speech signals.

The operation input signal, demodulated by the demodulating section 52, is inputted to the CPU 40. Under control of the CPU 40, the drive section 43 operates the lens motor 42 according to the operation input signal. This allows the director, staying in the direction room 12 distant from the examination room 10, is allowed to change the zoom magnification as to the endoscopic image. Incidentally, where split screen has been selected, the radio wave 76 is not sent even if the director operates the zooming button 100.

Here, in the case the identification signal on the RF signal, converted at the receiving section 51, has been superposed at the data superposing section 87, the electronic endoscope 14 controls the data superposing section 47 not to superpose a speech signal on the RF signal even if there is a speech input to the operator's microphone 17.

Meanwhile, where sending a radio wave 76 from the transmitting section 88 of the director's processor unit 70, when a speech is inputted to the director's microphone 72 and a speech signal is superposed on the radio wave 76 or the zooming button 100 is operated and an operation input signal is superposed on the radio wave 76, the speech-signal processing section 85 is controlled not to output the operator's speech through the director's speaker 74 even if a radio wave 20 superposed with a speech signal is received. Otherwise, detection is made as to whether or not an identification signal is superposed on the RF signal converted at the receiving section 83. In the case the identification signal has been superposed at the data superposing section 47, the transmitting section 88 is controlled not to send a radio wave even if the zooming button 100 is operated. This allows for sending/receiving a radio wave 20 superposed with a speech signal and a radio wave 76 superposed with speech and operation-input signals, according to half-duplex operation. Therefore, there is no need to prepare a frequency band separately from the radio waves 20, 76 in order to send/receive speech and operation-input signals. Thus, every signal can be sent and received in the common frequency band. This can architect an endoscopic diagnosing system 2 capable of using operator's endoscopic diagnosing units 11 greater in the number with assigned channels.

Although the embodiment allowed for speech communications at between the operator and the director, character input devices such as keyboards may be provided for the units 11, 13 so that the characters inputted on the character input device can be displayed on the monitor 16, 71. Meanwhile, exemplification was made to send/receive an operation input signal on the zooming button 100, the invention is not limited to it. The setting information may be provided for controlling the operation of the device, e.g. a receiving section of a radio wave 76 may be provided on the operator's processor unit 15, to send/receive various parameters (white balance, edge enhancement, etc.) for the video-signal processing section 67.

Incidentally, the director's monitor 71 may be structured with a touch panel, to enable the director's monitor 71 to perform the function as imposed upon the operation input device 75 in the embodiment. In such a case, full-screen display is satisfactorily switched to by touching an endoscopic image area where the director desires for full-screen display when display is based on split screens.

According to the endoscopic diagnosing unit of the invention, the director's processor unit has a transmitting section that modulates an operation input signal, inputted by the operation input device, into a radio wave to transmit; and a display control section that switches over between split-screen display to display endoscopic images separately between a plurality of electronic endoscopes on the director's monitor and full-screen display to display one endoscopic image entirely on the director's monitor. This can monitor the entire part panoramically, thus allowing the director to give a correct direction to the operators. Besides, by sending and receiving radio waves according to half-duplex operation, every signal can be sent and received in the common frequency band.

The entire disclosure of each and every foreign patent application from which the benefit of foreign priority has been claimed in the present application is incorporated herein by reference, as if fully set forth.

## Claims

1. An endoscopic diagnosing system that allows at least one operator who is present in at least one examination room to conduct endoscopic diagnosis, under instructions of a director who stays in a direction room, the system comprising:
(i) a plurality of operator's endoscopic diagnosing units in said at least one examination room, each of the operator's endoscopic diagnosing units including
an electronic endoscope that takes an image of a subject interior at a point-under-observation and outputs a video signal, to modulate the video signal into a first radio wave to-transmit,
an operator's processor unit that receives the first radio wave and demodulates the first radio wave into a video signal in a former form, to generate an endoscopic image from the video signal, and
an operator's monitor that displays the endoscopic image generated at the operator's processor unit; and
(ii) a director's endoscopic diagnosing unit in the direction room, the director's endoscopic diagnosing unit including
a director's processor unit that receives the first radio wave and demodulates the first radio wave into a video signal in a former form, to generate an endoscopic image from the video signal,
a director's monitor that displays the endoscopic image generated at the director's processor unit, and
an operation input device for the director to operate,
wherein the director's processor unit comprises:
a transmitting section that modulates an operation input signal, inputted by the operation input device, into a second radio wave to transmit; and
a display control section that switches display over between a split-screen display that displays endoscopic images of said plurality of electronic endoscopes on the director's monitor and a full-screen display that displays one of the endoscopic images entirely on the director's monitor:

2. An endoscopic diagnosing system according to claim 1,
wherein each of said plurality of operator's endoscopic diagnosing units further comprises:
an operator's information input device that inputs inquiry information of from the operator to the director; and
an operator's information output device that outputs instruction information of from the director to the operator, and
wherein the director's endoscopic diagnosing unit further comprises:
a director's information input device that inputs the instruction information; and
a director's information output device that outputs the inquiry information,
wherein the electronic endoscope superposes a signal representative of the inquiry information on the first radio wave which the video signal is modulated and then transmits the first radio wave,
the transmitting section modulates a signal representative of the instruction information into a third radio wave and then transmits the third radio, and
the electronic endoscope and the director's processor unit being to send and receive the first radio wave superposed with the signal representative of the inquiry information and the third radio wave which the signal representative of the instruction information is modulated, according to half-duplex operation.

3. An endoscopic diagnosing system according to claim 1,
wherein the director's processor unit comprises a receiving section that receives radio waves from said plurality of electronic endoscopes by a switchover at a constant time interval when the split-screen display is being done, and selectively receives a radio wave from one of said plurality of electronic endoscopes forming a basis of the one of endoscopic images being displayed on full screen when the full-screen display is being done.

4. An endoscopic diagnosing system according to claim 1,
wherein the operation input device comprises:
a display selecting section that selects an endoscopic image for the full-screen display out of the endoscopic images being displayed split on the screen, and
a display change section that changes between the split-screen display and the full-screen display.

5. An endoscopic diagnosing system according to claims 1,
wherein the operation input device comprises a zooming section that changes a zoom magnification as to the endoscopic image, and
the transmitting section modulates an operation input signal to the zooming section into the radio wave and transmits the radio wave.
